(19) Europäisches Patentamt
European Patent Office
Office européen des brevets

(11) EP 4 105 938 A1

(12) EUROPEAN PATENT APPLICATION

(43) Date of publication:
21.12.2022 Bulletin 2022/51

(21) Application number: 22178984.5

(22) Date of filing: 14.06.2022

(51) International Patent Classification (IPC):
G16H 20/17 (2018.01)    G16H 40/63 (2018.01)
A61B 5/00 (2006.01)    A61M 5/14 (2006.01)

(52) Cooperative Patent Classification (CPC):
G16H 20/17; A61M 5/14244; A61M 5/172;
G16H 40/63; A61B 5/4839

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 14.06.2021 US 202163210239 P

(71) Applicant: Insulet Corporation
Acton MA 01720 (US)

(72) Inventors:
• ZHENG, Yibin
  Hartland (US)
• LEE, Joon Bok
  Acton (US)
• ZADE, Ashutosh
  San Diego (US)
• O'CONNOR, Jason
  Acton (US)

(74) Representative: Peterreins Schley
Patent- und Rechtsanwälte PartG mbB
Hermann-Sack-Straße 3
80331 München (DE)

(54) **METHOD FOR MODIFICATION OF MAXIMUM DELIVERY LIMITS IN AUTOMATIC INSULIN DELIVERY SYSTEMS**

(57) The disclosed embodiments are directed to methods for adjusting, on a per individual basis, the maximum delivery limit of insulin delivered to a diabetic person via an automatic insulin delivery system, particularly for a diabetic who receives basal or bolus doses of insulin via an automatic insulin delivery system. Various embodiments of the method disclosed herein may be incorporated into a dosing algorithm of an automatic insulin delivery system.

FIG. 1

**Description**

**BACKGROUND**

For people with diabetes, all or a portion of their daily insulin requirements may be provided by an automatic insulin delivery system. For example, the diabetic person may manually provide bolus doses of insulin to compensate for increased blood glucose levels that result from ingestion of meals, while the basal requirements of the diabetic person may be provided by an automatic insulin delivery system that provides basal dosing of insulin at a predetermined basal rate (or vice versa).

The basal rate may be set by the diabetic person (or by a health care professional) and remains constant until it is changed. The automatic delivery system may be provided with an algorithm which periodically calculates how much insulin to deliver above or below the basal rate set by the diabetic person to compensate for excursions in the person's blood glucose level. The amount of insulin delivered above or below the basal rate is typically based on readings from a continuous glucose monitor, which provides periodic blood glucose level readings to the automatic insulin delivery system. For example, a continuous glucose monitor may supply blood glucose readings every five minutes, and the algorithm in the automatic insulin delivery system may be executed every five minutes to calculate how much insulin to deliver above or below the basal rate.

Automatic insulin delivery systems typically have maximum delivery limits which constrain the rate of the delivery of the basal insulin based on a generalized assessment of the robustness of the system against varying inaccuracies present in standard diabetes care. The maximum delivery limits exist for the safety of the diabetic person to prevent the automatic insulin delivery system from overreacting to increased blood glucose levels.

In some instances, the maximum delivery limit for basal insulin may be set to, for example, 4X the basal rate of the diabetic person. As an example, if the diabetic person is receiving insulin at a basal rate of 1.2 units per hour (0.1 units per five minutes), and the algorithm receives indications of a blood glucose excursion from the continuous glucose monitor, the additional insulin delivered could not exceed 0.4 units per five minutes (i.e., 4X the basal rate) due to the maximum delivery limit. However, the maximum delivery limit can often overly constrain or, in certain instances, under-constrain the automatic delivery of insulin for the diabetic person. As such, the ability to adjust the maximum delivery limit on a per individual basis would be beneficial. Therefore, it would be desirable to provide a method, incorporated into the algorithm for the automatic insulin delivery system, to allow the maximum delivery limit to be adjusted for individual diabetic persons based on various criteria.

**DEFINITIONS**

As used herein, the terms *"user"* and *"person"* are used interchangeably and are meant to include persons suffering from diabetes who are using the disclosed invention to control basal or bolus dosing of insulin.

**SUMMARY**

This Summary is provided to introduce a selection of concepts in a simplified form that are further described below in the Detailed Description. This Summary is not intended to identify key or essential features of the claimed subject matter, nor is it intended as an aid in determining the scope of the claimed subject matter.

Disclosed herein are methods for adjusting, on a per individual basis, the maximum delivery limit of insulin delivered to a diabetic person via an automatic insulin delivery system, particularly for a diabetic person who receives basal doses of insulin via an automatic insulin delivery system but provides bolus doses of insulin manually (or vice versa).

In a first embodiment of the invention, the maximum delivery limit may be adjusted based on the percentage of the cycles (i.e. each time the automatic insulin delivery algorithm is executed) that the delivery of insulin was limited by the maximum delivery limit.

In a second embodiment of the invention, the maximum delivery limit may be adjusted based on a standard deviation of blood glucose levels of the diabetic person. (i.e. how widely the blood glucose readings deviate from the mean). A wider standard deviation indicates that person's blood glucose levels are not well-controlled, and that the maximum delivery limit may need to be raised.

In the third embodiment of the invention the modification of the maximum delivery limit may be based on the percentage of cycles wherein the person's blood glucose readings indicated hypoglycemia.

The fourth embodiment of the invention assumes a separate nighttime maximum delivery limit for use during nighttime periods which may be different from the maximum delivery limit used during the day. In this embodiment of the invention, the nighttime maximum delivery limit can be adjusted for individuals based on the percentage of low overnight blood glucose levels experienced by the diabetic person.

In preferred embodiments of the invention, the maximum delivery limits may be modified by modifying a factor by which the diabetic person's basal rate is multiplied to calculate the maximum delivery limit. For example, if the maximum delivery limit is currently 4X the person's basal rate, the maximum delivery limit may be raised to 5X the person's basal rate.

Although exemplary embodiments are described herein as a drug delivery device that administers insulin to a user, the present invention is not limited to the use of insulin. By way of example, other types of drugs that may be administered include (but are not limited to) pramlintide and glucagon-like peptide 1 (GLP-1).

Preferred embodiments of the invention may comprise any combination of the various embodiments discussed above.

## BRIEF DESCRIPTION OF THE DRAWINGS

To easily identify the discussion of any particular element, the most significant digit or digits in a reference number refer to the figure number in which that element is first introduced.

FIG. 1 is a block diagram of an AID system including the basal dosing application of which present invention may be a part.

FIG. 2 is a flowchart showing an overall process for adjusting the maximum delivery limit for the current cycle of the basal delivery algorithm.

FIG. 3 is a flowchart showing a process for calculating an adjustment factor used to adjust the maximum delivery limit in accordance with a first embodiment of the invention.

FIG. 4 is a flowchart showing a process for calculating an adjustment factor used to adjust the maximum delivery limit in accordance with a second embodiment of the invention.

FIG. 5 is a flowchart showing a process for calculating an adjustment factor used to adjust the maximum delivery limit in accordance with a third embodiment of the invention.

FIG. 6 is a flowchart showing a process for calculating a nighttime maximum delivery limit in accordance with a fourth embodiment of the invention

## DETAILED DESCRIPTION

Methods in accordance with the present disclosure will now be described more fully with reference to the accompanying drawings, where one or more embodiments or various aspects of the invention are shown. The methods may be embodied in many different forms and are not to be construed as being limited to the embodiments set forth herein. Instead, these embodiments are provided so the disclosure will be thorough and complete, and will fully convey the scope of the systems and methods to those skilled in the art. Each of the methods disclosed herein provides one or more advantages over conventional systems and methods.

Persons suffering from diabetes may have all or a portion of their daily insulin requirements delivered via an automatic insulin delivery system. As an example, an automatic insulin delivery system may provide the person's basal dosing requirements, while the person's bolus dosing requirements are delivered manually by the person. In such cases, the person, in consultation with a healthcare professional, may set the basal rate or, alternatively, the person may be provided with an automatic insulin delivery system having the required basal rate set as a pre-programed parameter. The automatic insulin delivery system may continuously deliver insulin to the person at the specified basal rate, for example, in discrete periodic increments.

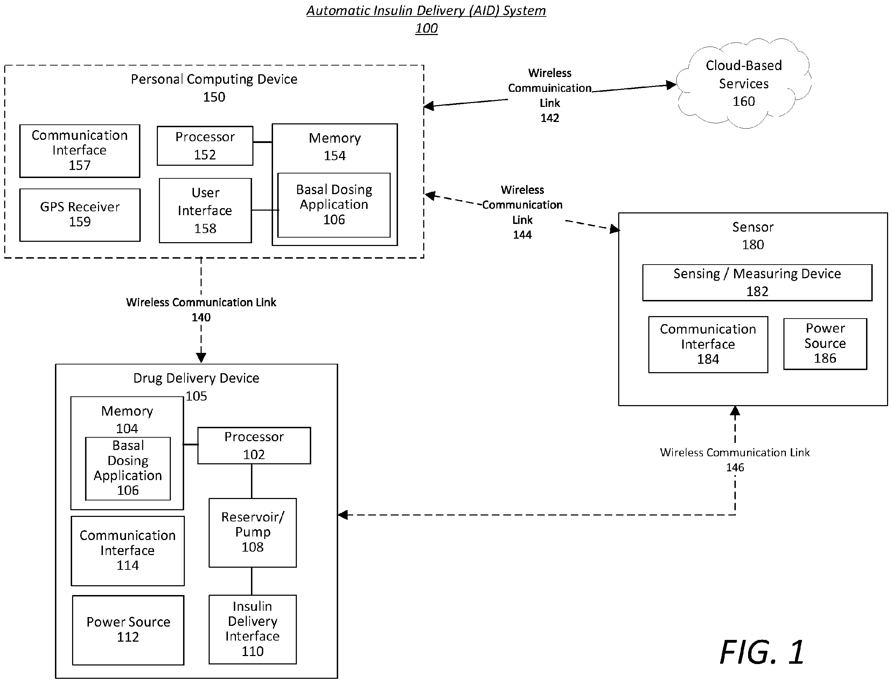

FIG. 1 is a block diagram showing an automatic insulin delivery (AID) system 100 of the type in which the various embodiments of the present invention may be implemented. The AID system 100 includes a basal dosing application 106 for calculating the basal dosing requirements of the person and for controlling the mechanical aspects of a drug delivery device 105 responsible for the actual delivery of the insulin to the user. The basal dosing application 106 may implement the various novel embodiments of the invention described in the Summary above and in more detail below.

In a primary embodiment of the invention, the basal dosing application 106 may be implemented as a software application executing on a processor in a drug delivery device 105 as part of an AID system 100. Drug delivery device 105 may be, for example, a wearable drug delivery device which is discreetly disposed on the body of the user and held in place on the user's skin by an adhesive. Alternatively, drug delivery device 105 may be, for example, a drug delivery device carried by a user (e.g., on a belt or in a pocket) and having an infusion set with tubing connecting the drug delivery device 105 to a cannula that penetrates the user's skin.

Drug delivery device 105 may include a processor 102 in communication with memory 104 containing basal dosing application 106 which is executed by processor 102. In accordance with the basal dosing application 106, processor 102 may control a reservoir/pump 108 suitable for delivering insulin to the user via insulin delivery interface 110 which may be, for example, a subcutaneous cannula extending from one or more housings of drug delivery device 105 and into the body of the user. Drug delivery device 105 may further include a wireless communication interface 104. The overall drug delivery device 105 may be powered by power source 112, which may be, for example, batteries or a power harvesting apparatus.

AID system 100 may further include a sensor 180 which may comprise a sensing / measuring device 182 which may be, for example, a continuous glucose monitor (CGM). Like drug delivery device 105, sensor 180 may also be a wearable device disposed on the body of the user. Sensor 180 may include power source 186 and a wireless communication interface 184.

Drug delivery device 105 and sensor 180 may communicate with each other via wireless communication link 146. Sensor 180 may provide periodic readings of the blood glucose level of the user to drug delivery device 105 via wireless communication link 146. For example, sensor 180, in one embodiment, may provide blood glucose level readings to drug delivery device 105 every 5 minutes. Other intervals for reporting the blood glucose levels of the user are within the scope of the invention. In some embodiments of the present invention, the periodic readings of the blood glucose level of the user may be used, as described below, to modify the maximum delivery limit for one or more cycles of the execution of basal dosing application 106.

In alternate embodiments, basal dosing application 106 may execute on a personal computing device 150 instead of on drug delivery device 105 and may communicate the basal dosing requirements to drug delivery device 105 via wireless communication link 140. Personal computing device 150 may be configured with a processor 152 and a memory 154 containing the basal dosing application 106. Personal computing device 150 may be further configured with a user interface 158 which may be used by the basal dosing application 106 to enable interaction with a user.

In some embodiments, personal computing device 150 may comprise, for example, a smartphone, a tablet device, a smartwatch, a dedicated personal diabetes manager, or any other personal mobile computing device capable of running basal dosing application 106 and communicating with the drug delivery device 105, cloud-based services 160 and sensor 180 via any well-known wireless communication protocol.

Basal dosing application 106 may have a limited user interface which may be used, for example, to input the user's basal rate and/or to provide feedback to the user regarding the delivery of the insulin and the sensed blood glucose level readings. In some embodiments, the blood glucose level readings may be received from sensor 180, directly via communication link 144 or indirectly via drug delivery device 105 via wireless communication link 140.

In alternative embodiments wherein no sensor 180 is used, the user may manually take blood glucose readings and enter the blood glucose readings directly via user interface 158. The blood glucose readings may thereafter be transmitted to drug delivery device 105 via wireless communication link 140 for use by basal dosing application 106. In such cases, a limited front-end user interface may be provided on personal computing device 150 for the purpose of allowing the entry of blood glucose readings.

In some embodiments, the functionality of basal dosing application 106 may be split between personal computing device 150 and drug delivery device 105 in any convenient manner.

Basal dosing application 106 may further communicate with cloud-based services 160 via communication link 142. In alternative embodiments of the invention, the basal dosing application 106 may be implemented in a cloud-based service 160 and accessed via wireless communication link 142 with the user's personal computing device 150.

As previously discussed, basal dosing application 106 may control the delivery of basal doses of insulin to the user at periodic intervals based on the specified basal rate. However, under certain circumstances, for example, should the basal dosing application 106 receive an indication from sensor 180 of an excursion in the user's blood glucose level, additional insulin may be delivered over a period of several cycles to address the excursion.

Basal dosing application 106 may comprise an algorithm which periodically execute in cycles. For example, a cycle may comprise a 5-minute period during which a new blood glucose level reading may be received from sensor 180. The algorithm may determine, in response to each additional blood glucose level reading, if additional insulin is required above and beyond the insulin that is indicated by the basal rate and may control the delivery of the additional insulin during the current cycle. This additional insulin may be delivered as micro-boluses, which are insulin amounts above the baseline basal rate. Accordingly, the basal rate may not change, but additional insulin may be delivered together with the basal insulin as micro-boluses. This may repeat indefinitely or in subsequent cycles until the blood glucose readings of the user return to an acceptable level.

For the safety of the user, the maximum amount of insulin which may be delivered during any given cycle may be limited by a maximum delivery limit. Eq. (1) below shows, for each algorithmic cycle $i$, the amount of insulin to be delivered by the AID system ($I_{AID}$):

$$I_{AID}(i) = \min(I_{calc}(i), b_{max}(i))$$

$$(1)$$

where:

$I_{calc}(i)$ is the quantity of insulin as calculated by the basal dosing application required during the current cycle to address the current blood glucose level of the user; and

$b_{max}(i)$ is the maximum delivery limit for the current cycle, which may be calculated as the basal rate multiplied by a factor, as shown in Eq. (2):

$$b_{max} = M \cdot R_{basal}$$

$$(2)$$

where:

$R_{basal}$ is the basal rate; and
$M$ is the factor by which the basal rate is multiplied.

In some AID systems, the factor $M$ may be hardcoded to a value of 4, such that the maximum delivery limit for each cycle is 4X the basal rate ($R_{basal}$). However, in some instances, the maximum delivery limit may be determined, over time, to be insufficient to address excursions in the blood glucose level of the user. In such cases, and, in accordance with the present invention, the maximum delivery limit may be adjusted for each cycle by adjusting the factor $M$ for each cycle $i$ such that the maximum delivery limit may be calculated by the equation:

$$b_{max}(i) = M(i) \cdot R_{basal}$$

$$(3)$$

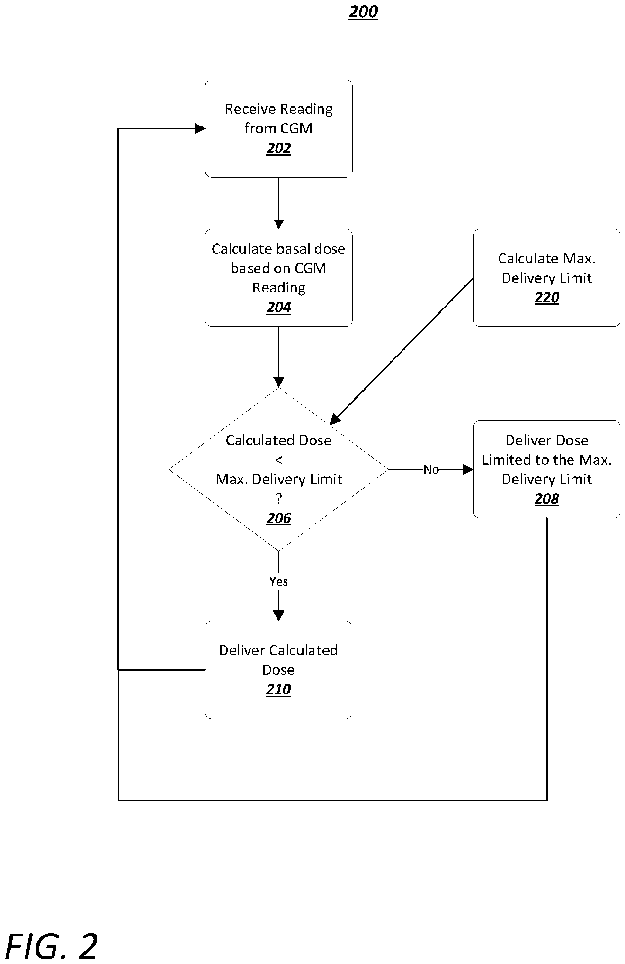

The process for adjusting the total amount of insulin delivered by the AID system 100 for each cycle of the algorithm is shown in **FIG. 2**. At 202, the basal dosing application 106 receives a blood glucose level reading from sensor 180. In accordance with the basal dosing algorithm 106, a basal dose is calculated at 204 as required to address the current blood glucose level reading. At 220, a maximum delivery limit for a bolus dose is calculated in accordance with various embodiments of the present invention. The process then determines, at 206, if the calculated basal dose is less than the maximum delivery limit. That is, the amount of glucose present in the user's blood stream may be addressed by a dose of insulin which is less than the maximum delivery limit. If this is the case, the calculated basal dose of insulin is delivered at 210 as one or more micro-boluses. However, if the amount of glucose present in the user's bloodstream requires a dose of insulin larger than the calculated maximum delivery limit, a basal dose limited by the maximum delivery limit is delivered at 208 as one or more micro-boluses.

Note that, although the required quantity of insulin may be calculated for each cycle, the actual delivery of the insulin to the user may not occur during every cycle of the algorithm. In some embodiments, the granularity of the doses that are able to be delivered by the drug delivery device 105 may be limited by the physical properties of the device and, as such, actual delivery of the insulin may occur when the accumulated insulin doses from each cycle reaches a particular threshold.

In accordance with various exemplary embodiments of the present invention, the factor $M$ by which the basal rate $R_{basal}$ is multiplied to obtain the maximum delivery limit for each cycle may, in one embodiment, be calculated by Eq. (4):

$$M_{new} = M_{current} \cdot (1 - 0.2N) + AF \cdot (0.2N)$$

(4)

where:

$M_{current}$ is the current value of the factor $M$;

$AF$ is an adjustment factor which may be calculated in various different ways in accordance with various embodiments of the invention; and

$N$ is the current number of days in the current pump session, over which the calculation to modify $M$ is carried out.

As would be realized by one of skill in the art, the invention is not meant to be limited to embodiments in which the new value for the factor $M$ is calculated using the exact factors shown in Eq. (4). In other embodiments of the invention, meant to be included within the scope of the present invention, new values for the factor $M$ may be calculated in various different ways.

In some embodiments, it is desirable that the new value for the factor $M$ be limited in a range between an $M_{low}$ value and an $M_{high}$ value such that the maximum delivery limit may also be limited to a specific range. For instance, in some embodiments of the invention, the range for the factor $M$ may be set between a low value of 3 and a high value of 8. In other embodiments, the low value and high value may be set differently. In some embodiments, the factor $M$ may start at a predetermined initial value and may be adjusted therefrom. In some embodiments, the initial value for the factor $M$ may be 4. In other embodiments, the factor $M$ may start at initial value which is dependent upon previous adjustments from previous pump sessions. In some embodiments, the factor $M$ may be reset to an initial value at the start of each pump session.

The factor $M$ may be adjusted by calculating a new adjustment factor $AF$ and applying the new adjustment factor in accordance with Eq. (4), or in accordance with any other equation contemplated for the adjustment of the factor $M$.

In a first embodiment of the invention, the maximum delivery limit is based on the percentage of cycles the basal dose of insulin delivered to the user was limited by the maximum delivery limit. The percentage of cycles may be calculated over a past predetermined number of cycles which may be, for example, the cycles executed during the current pump session. If the percentage is high, the adjustment factor may be calculated such as to increase the value of the factor $M$, and, as a result, increasing the maximum delivery limit, while, if the percentage is low, the adjustment factor may be calculated such as to decrease the value of the factor $M$ and, as a result, decreasing the maximum delivery limit.

In an exemplary implementation of the first embodiment, the adjustment factor $AF$ may be adjusted in accordance with Eq. (5):

$$AF = min\left( M_{high}, max\left( M_{low}, \frac{N_{con}/N_{all}}{0.015} \right) \right)$$

(5)

where,

$N_{con}$ is the number of cycles in the past predetermined number of cycles in which the delivered dose of insulin was limited by the maximum delivery limit;

$N_{all}$ is the total number of past predetermined cycles over which the percentage is calculated; and

the constant "0.015" is a heuristic value that was derived empirically and which may be varied in other embodiments of the invention.

Once the adjustment factor $AF$ has been calculated, it may be used in Eq. (4) to calculate the new value for the factor $M$ for each cycle. One other factor that may be considered in the calculation of the adjustment factor is that, under typical system use, the user may be constrained to the maximum delivery limit approximately 6% of the time. This may be incorporated as a tunable parameter in the calculation operating between a low and a high value, for example, in a range between 2% and 10%.

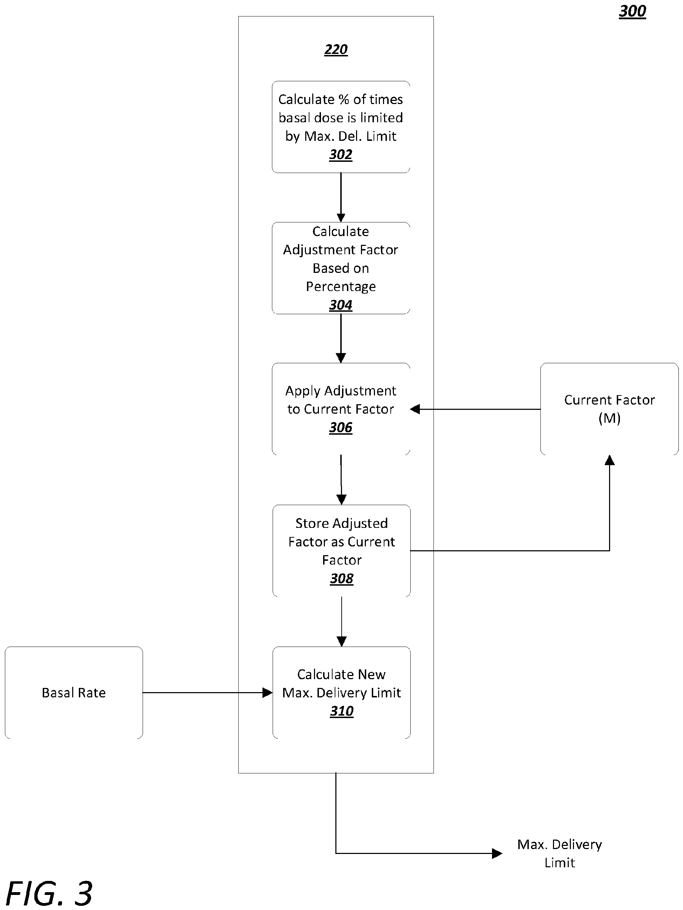

The process 220 for calculating a maximum delivery limit in accordance with the first embodiment of the invention is shown as process 300 in **FIG. 3.** At 302, the percentage of cycles ($N_{con}/N_{all}$) that the calculated basal dose has been limited by the maximum delivery limit is calculated. The adjustment factor $AF$ is then calculated at 304 in accordance with Eq. (5), based on the percentage calculated in step 302. At 306, current factor ($M_{current}$) is retrieved and is adjusted using the adjustment factor as applied to the current factor in accordance with Eq. (4), resulting in a new value of the factor $M_{new}$. At step 308, the new value of the factor $M_{new}$ is stored as the current factor for the next cycle. At step 310, the basal rate is retrieved and the new maximum delivery limit for the current cycle, $b_{max}(i)$, is calculated as the product of the new factor $M_{new}$ and the basal rate.

In a second embodiment of the invention, the maximum delivery limit is based on the standard deviation of a set of blood glucose readings provided by sensor 180. In one embodiment, the set of blood glucose readings may comprise all blood glucose readings from a past, predetermined period of time, for example, all blood glucose readings from the current pump session (e.g., over the past 72 hours). In one embodiment, a standard deviation that deviates significantly from the mean of the set of blood glucose readings would indicate that the blood glucose levels of the user are not well-controlled and that the maximum delivery limit may be raised.

In an exemplary implementation of the second embodiment, the adjustment factor $AF$ may be adjusted in accordance with Eq. (6):

$$AF = min\left(M_{high}, max\left(M_{low}, \frac{std\,(BG_{prev})}{11.25}\right)\right)$$

$$(6)$$

where,

$BG_{prev}$ is the set of blood glucose readings upon which the standard deviation is calculated. In preferred embodiments of the invention, each new blood glucose reading received from sensor 180 is added to the set of blood glucose readings and the standard deviation is recalculated during each cycle of the algorithm.

The constant ("11.25") in Eq. (6), is provided under the assumption that the typical standard deviation of blood glucose readings for users with diabetes is approximately 45 mg/dL and is based on a baseline adjustment factor of 4 (i.e., 45 / 11.25 = 4). Other constants could be used. By adjusting the constant in Eq. (6), the width of the typical standard deviation may be varied and, as a consequence, the adjustment factor may be more or less affected by the standard deviation of the set of blood glucose readings. In preferred embodiments, the width of the typical standard deviation may be tunable from 35 md/dL to 55 mg/dL.

Once the adjustment factor $AF$ has been calculated based on Eq. (6), it may be used in Eq. (4) to calculate the new value for the factor $M$ in accordance with the second embodiment of the invention.

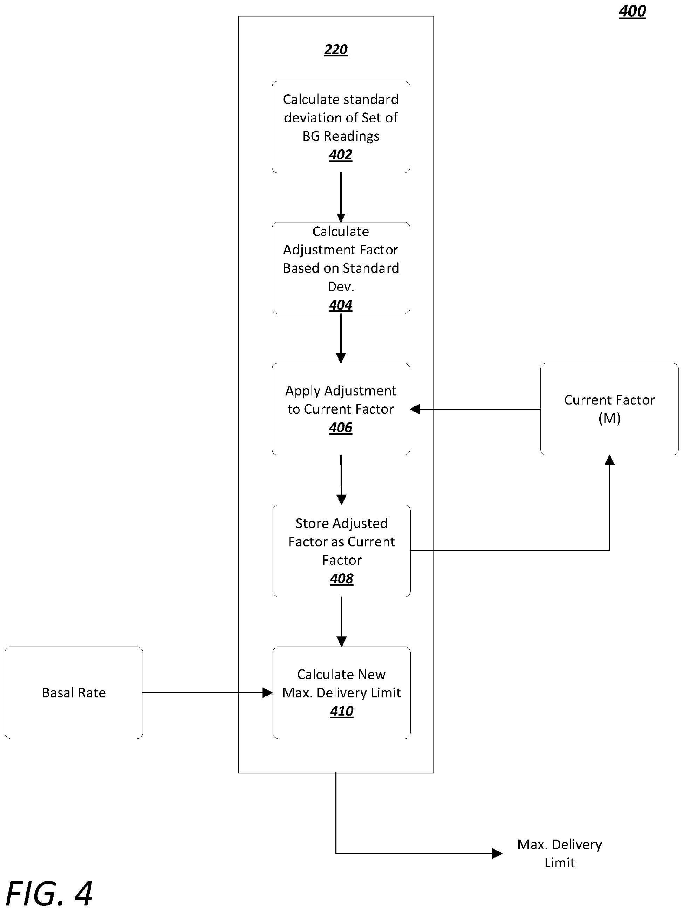

The process 220 for calculating a maximum delivery limit in accordance with the second embodiment of the invention is shown as process 400 in **FIG. 4.** At 402, the most recent blood glucose reading is added to the set of blood glucose readings and the standard deviation of the set of blood glucose readings is calculated. The adjustment factor $AF$ is then calculated at 404 in accordance with Eq. (6), based on the standard deviation calculated in step 402. At 406, the current factor ($M_{current}$) is retrieved and is adjusted using the adjustment factor as applied to the current factor in accordance with Eq. (4), resulting in a new value of the factor ($M_{new}$). At step 408, the new value of the factor ($M_{new}$) is stored as the current factor for the next cycle. At step 410, the basal rate is retrieved and the new maximum delivery limit for the current cycle, $b_{max}(i)$, is calculated as the product of the new factor $M_{new}$ and the basal rate.

In a third embodiment of the invention, the maximum delivery limit is based on a percentage of cycles wherein the received blood glucose reading indicates hypoglycemia. The percentage of cycles may be calculated over a past predetermined number of cycles which may be, for example, the cycles executed during the current pump session. In this embodiment, if the percentage is high, the adjustment factor may be calculated such as to decrease the value of the factor $M$, thus decreasing the maximum delivery limit, while, if the percentage is low, the adjustment factor may be calculated such as to increase the value of the factor $M$, thus increasing the maximum delivery limit.

In an exemplary implementation of the third embodiment, the adjustment factor $AF$ may be adjusted in accordance with Eq. (7):

$$AF = min \left( M_{high}, max \left( M_{low}, \frac{0.06}{N_{hypo} / N_{all}} \right) \right)$$

$$(7)$$

where,

$N_{hypo}$ is the number of cycles in the past predetermined number of cycles in which the received blood glucose reading indicates hypoglycemia; and

$N_{all}$ is the total past predetermined number of cycles over which the percentage is calculated.

In preferred embodiments of the invention, hypoglycemia is indicated when the blood glucose reading is below 70 mg/dL. In other embodiments, the threshold for determining whether the blood glucose reading indicated hypoglycemia may be higher or lower than 70 mg/dL.

Under typical circumstances, the hypoglycemic incidents for a diabetic person will not be greater than approximately 1.5%, which Eq. (7) takes into account via the constant ("0.06") and an adjustment factor of 4 (i.e., 0.06 / 0.015 = 4). In other embodiments of the invention, the constant may be adjusted to account for typical instances of hypoglycemia in the range of 1% to 2%.

Once the adjustment factor $AF$ has been calculated using Eq. (7), it may be used in Eq. (4) to calculate the new value for the factor $M$ in accordance with the third embodiment of the invention.

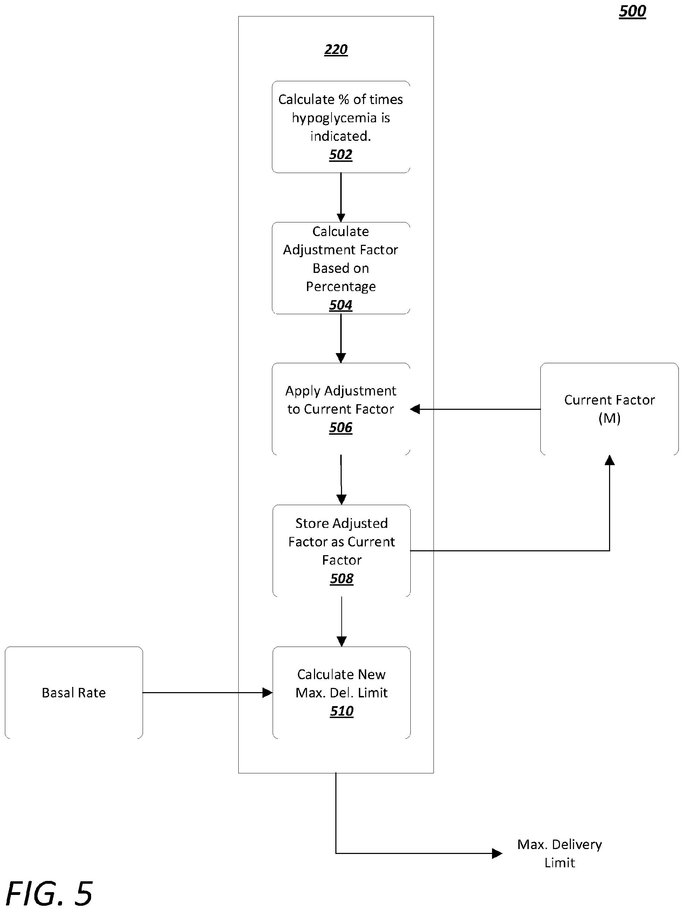

The process 220 for calculating a maximum delivery limit in accordance with the third embodiment of the invention is shown as process 500 in **FIG. 5.** At 502, the percentage of cycles wherein the blood glucose reading indicates hypoglycemia ($N_{hypo}/N_{all}$) is calculated. The adjustment factor $AF$ is then calculated at 504 in accordance with Eq. (7), based on the percentage calculated in step 502. At 506, current factor ($M_{current}$) is retrieved and is adjusted using the adjustment factor as applied to the current factor in accordance with Eq. (4), resulting in a new value of the factor $M_{new}$. At step 508, the new value of the factor $M_{new}$ is stored as the current factor for the next cycle. At step 510, the basal rate is retrieved and the new maximum delivery limit for the current cycle, $b_{max}(i)$, is calculated as the product of the new factor $M_{new}$ and the basal rate.

In a fourth embodiment of the invention, a separate nighttime maximum delivery limit may be derived for the user to be applied during the night. In this embodiment, "night" may be defined in any convenient way. For example, the night may be a period of time beginning after a post-prandial window following the evening meal and ending when the user consumes breakfast. Alternatively, for example, night may be defined as period of time during which the user is sleeping, or the time during which a typical user would be sleeping (e.g., 10:30pm - 6:30am). However, the definition of night is not meant to be limited to these examples. Regardless of the specific definition of night, a basal dosing application 106 implementing this embodiment of the invention should check, during each cycle, whether the nighttime criteria have been met.

The adjustment of the nighttime maximum delivery limit, $b_{night}(i)$, is meant to compensate for expected generally lower blood glucose levels during the period when the user is either inactive or not consuming anything that would add glucose to the user's blood stream. Similar to Eq. (2) for the adjustment of the maximum delivery limit for the daytime, the nighttime maximum delivery limit is defined for each cycle i by the equation:

$$b_{night}(i) = M_{night}(i) \cdot R_{basal}$$

$$(8)$$

The adjustment of the nighttime maximum delivery limit may be triggered upon the satisfaction of one or more conditions, indicating that the user has experienced a low blood glucose reading during a nighttime period a higher percentage of the time than is normally expected. In this embodiment, the blood glucose reading could be considered low if it falls below a predetermined threshold. In a preferred embodiment of the invention, the threshold below which the blood glucose reading is considered low is 60 ml/dL, but the invention is not meant to be limited to that value and may be set higher or lower on alternate embodiments.

The calculation of the adjustment of the nighttime maximum delivery limit may be triggered by the satisfaction of one or more conditions. In various embodiments of the invention, the calculation may be triggered when at least one of several possible conditions are met. In other embodiments of the invention, the calculations may be triggered when all specified conditions are met.

In one embodiment of the invention, a first condition may be one or more blood glucose readings below the predetermined low threshold for two consecutive nights. In one embodiment of the invention, a second condition may be one or more blood glucose readings below the predetermined low threshold three out of the last seven nights. As such, the calculation may be triggered, for example, by a TRUE result of a Boolean expression such as:

$(condition_1$ OR $condition_2)$

or, in other embodiments,

$$(condition_1 \text{ AND } condition_2)$$

$$(9)$$

As should be realized, any combination of conditions may be used to trigger the calculation of the nighttime maximum delivery limit, and the invention is not meant to be limited by the example conditions provided.

Once it has been determined that the nighttime maximum delivery limit should be recalculated, the factor $M_{night}(i)$ may be calculated by the equation:

$$M_{night}(i) = \min\left(M_{new}(i), mean\left(M_{new(not\ low)}\right)\right)$$

$$(10)$$

where:

$M_{new}(i)$ can be calculated as set forth in any one of the first three embodiments of the invention; and
$mean(M_{new(not\ low)})$ is the mean value of all of the $M_{new}$ readings for a past predetermined number of nights when there was no blood glucose reading received below the low threshold.

As an example, if the calculation of the nighttime maximum delivery limit is triggered by blood glucose readings below the low threshold in three out of the last seven nights, the mean value will be calculated over all of the $M_{new}$ values for the four nights wherein no blood glucose reading below the low threshold was received.

It is contemplated that, in this embodiment of the invention, the nighttime maximum delivery limit would revert to the daytime maximum delivery limit, as calculated in accordance with any of the first three embodiments of the invention, or in any other way, when the nighttime period has ended. That is, the nighttime maximum delivery limit would only be applied during the nighttime period.

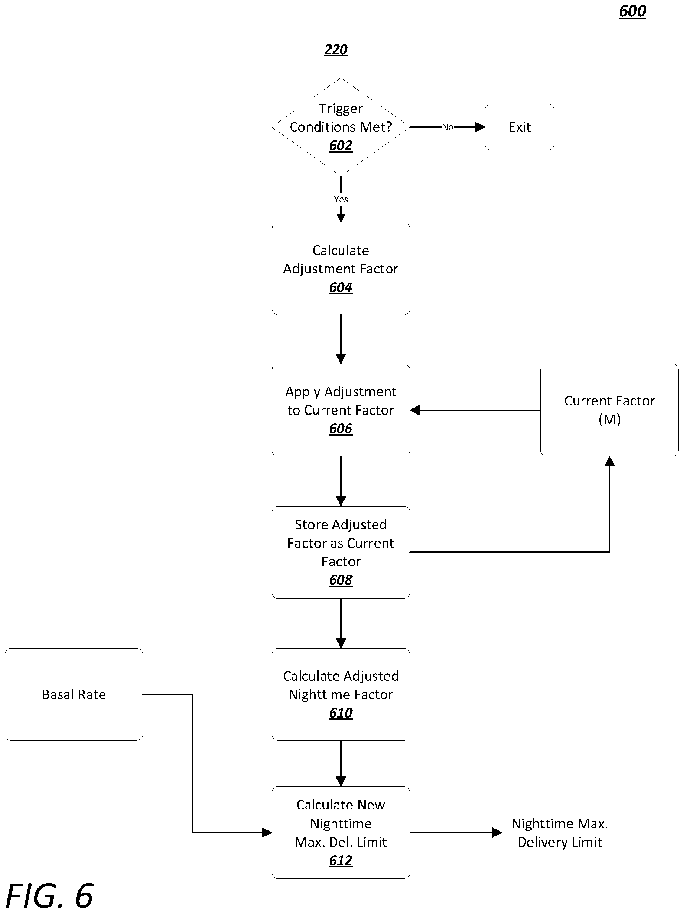

The process 600 for calculating the nighttime maximum delivery limit is shown in flowchart form in **FIG. 6.** At 602, it is determined if the trigger conditions indicating nighttime have been met. If not, the process exits. If the trigger conditions are met at 602, the process continues to step 604 wherein the adjustment factor is calculated, as in the first three embodiments of the invention. At 606, the current factor is retrieved and the adjustment is applied to the current factor. At 608, the adjusted factor is stored as the current factor for the next iteration of the process. At 610, an adjusted nighttime factor is calculated using Eq. (10). As previously discussed, the adjusted nighttime factor may be calculated as the minimum of the $M_{new}$ and the mean of all $M_{new}$ factors for a predetermined number of nights wherein no blood glucose level below the low threshold was received. In other embodiments, other criteria may be applied for calculating the adjusted nighttime factor $M_{night}$. At 612, the new nighttime maximum delivery limit is calculated as a product of the basal rate and the adjusted nighttime factor. The new nighttime maximum delivery limit is used as the maximum delivery limit at decision point 206 in **FIG. 2** during nighttime periods, where it is determined if the dose to be delivered to the user should be the calculated dose or the nighttime maximum delivery limit, if the calculated basal dose is greater than the nighttime maximum delivery limit.

In various implementations of the invention, any of the four embodiments discussed herein may be used alone or in any combination. The algorithm executing as part of basal dosing application 106 may implement, during each cycle, the criteria for altering the maximum delivery limit specified by any one or any combination of the embodiments discussed herein.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:

1. A method comprising:
executing a basal dosing algorithm, wherein each cycle of the algorithm comprises:

receiving a blood glucose reading;
determining a required basal dose based on the received blood glucose reading;
adjusting a maximum delivery limit; and
determining a basal dose to be delivered, the basal dose comprising the lesser of the required basal dose and a dose limited by the maximum delivery limit.

2. The method of embodiment 1 further comprising:
delivering the basal dose as one or more micro-boluses.

3. The method according to any one of the preceding embodiments wherein the maximum delivery limit is constrained between a lower limit and an upper limit.

4. The method according to any one of the preceding embodiments wherein adjusting the maximum delivery limit comprises:

calculating a percentage of time during a past predetermined time period that delivery of insulin had been limited by the maximum delivery limit; and
adjusting the maximum delivery limit based on the calculated percentage.

5. The method according to any one of the preceding embodiments wherein the adjusted maximum delivery limit is a product of a factor and a basal rate and further wherein the factor is a function of the calculated percentage and a current maximum delivery limit.

6. The method according to any one of the preceding embodiments wherein the percentage of time during a past predetermined time period that delivery of insulin has been limited by the maximum delivery limit is calculated as a percentage of cycles of the basal dosing algorithm.

7. The method according to any one of the preceding embodiments wherein a higher percentage indicates an upward adjustment of the maximum delivery limit and a lower percentage indicates a downward adjustment of the maximum delivery limit.

8. The method according to any one of the preceding embodiments wherein adjusting the maximum delivery limit comprises:

adding the received blood glucose reading to a set of blood glucose reading from a past predetermined time period;
calculating a standard deviation of the set of blood glucose readings;
adjusting the maximum delivery limit based on the calculated standard deviation.

9. The method according to any one of the preceding embodiments wherein the adjusted maximum delivery limit is a product of a factor and a basal rate and further wherein the factor is a function of the calculated standard deviation and a current maximum delivery limit.

10. The method according to any one of the preceding embodiments wherein the standard deviation of the set of blood glucose readings is calculated every cycle of the basal dosing algorithm.

11. The method according to any one of the preceding embodiments wherein a wider standard deviation indicates an upward adjustment of the maximum delivery limit and a narrower standard deviation indicates a downward adjustment of the maximum delivery limit.

12. The method according to any one of the preceding embodiments wherein adjusting the maximum delivery limit comprises:

    calculating a percentage of time during a past predetermined time period that a blood glucose reading has indicated hypoglycemia; and
    adjusting the maximum delivery limit based on the calculated percentage.

13. The method according to any one of the preceding embodiments wherein the adjusted maximum delivery limit is a product of a factor and a basal rate and further wherein the factor is a function of the calculated percentage and a current maximum delivery limit.

14. The method according to any one of the preceding embodiments wherein the percentage of time during a past predetermined time period that a blood glucose reading has indicated hypoglycemia is calculated as a percentage of cycles of the basal dosing algorithm.

15. The method according to any one of the preceding embodiments wherein a higher percentage indicates a downward adjustment of the maximum delivery limit and a lower percentage indicates an upward adjustment of the maximum delivery limit.

16. A method for determining a nighttime maximum delivery limit for use during a nighttime period comprising:

    applying a nighttime maximum delivery limit during a nighttime period; and
    applying a daytime maximum delivery limit during a non-nighttime period.

17. The method of embodiment 16, further comprising:

    determining that a blood glucose reading below a low threshold has been received during one or more nighttime periods of a predetermined number of past nighttime periods;
    determining that the number of blood glucose readings below a low threshold satisfy one or more triggering conditions for triggering the calculation of a new nighttime maximum delivery limit; and
    adjusting the nighttime maximum delivery limit.

18. The method of any one of embodiments 16 to 17 wherein a first triggering condition comprises receiving a blood glucose reading below a low threshold on two consecutive nights.

19. The method of any one of embodiments 16 to 18 wherein a second triggering condition comprises receiving a blood glucose reading below a low threshold on three nights of the past seven nights.

20. The method of any one of embodiments 16 to 19 wherein the nighttime maximum delivery limit comprises a product of a basal rate and a factor, the factor being the lesser of a current factor or a factor calculated as the mean of factors calculated on a predetermined number of past nights wherein no blood close glucose reading below the low threshold has been received.

21. The method of any one of the preceding embodiments, wherein the method is implemented as a software application to be executed on a processor in a drug delivery device.

**Claims**

1. A method implemented as a software application to be executed on a processor, in particular on a processor of a drug delivery device, comprising:
   executing a basal dosing algorithm, wherein each cycle of the algorithm comprises:

receiving a blood glucose reading;
determining a required basal dose based on the received blood glucose reading;
adjusting a maximum delivery limit; and
determining a basal dose to be delivered, the basal dose comprising the lesser of the required basal dose and a dose limited by the maximum delivery limit.

2. The method of claim 1 further comprising:
delivering the basal dose as one or more micro-boluses.

3. The method of any one of the preceding claims wherein the maximum delivery limit is constrained between a lower limit and an upper limit.

4. The method of any one of the preceding claims wherein adjusting the maximum delivery limit comprises:

calculating a percentage of time during a past predetermined time period that delivery of insulin had been limited by the maximum delivery limit; and
adjusting the maximum delivery limit based on the calculated percentage.

5. The method of any one of the preceding claims wherein the adjusted maximum delivery limit is a product of a factor and a basal rate and further wherein the factor is a function of the calculated percentage and a current maximum delivery limit.

6. The method of any one of the preceding claims wherein the percentage of time during a past predetermined time period that delivery of insulin has been limited by the maximum delivery limit is calculated as a percentage of cycles of the basal dosing algorithm.

7. The method of any one of the preceding claims wherein a higher percentage indicates an upward adjustment of the maximum delivery limit and a lower percentage indicates a downward adjustment of the maximum delivery limit.

8. The method of any one of the preceding claims wherein adjusting the maximum delivery limit comprises:

adding the received blood glucose reading to a set of blood glucose reading from a past predetermined time period;
calculating a standard deviation of the set of blood glucose readings;
adjusting the maximum delivery limit based on the calculated standard deviation.

9. The method of any one of the preceding claims wherein the adjusted maximum delivery limit is a product of a factor and a basal rate and further wherein the factor is a function of the calculated standard deviation and a current maximum delivery limit.

10. The method of any one of the preceding claims wherein the standard deviation of the set of blood glucose readings is calculated every cycle of the basal dosing algorithm.

11. The method of any one of the preceding claims wherein a wider standard deviation indicates an upward adjustment of the maximum delivery limit and a narrower standard deviation indicates a downward adjustment of the maximum delivery limit.

12. The method of any one of the preceding claims wherein adjusting the maximum delivery limit comprises:

calculating a percentage of time during a past predetermined time period that a blood glucose reading has indicated hypoglycemia; and
adjusting the maximum delivery limit based on the calculated percentage.

13. The method of any one of the preceding claims wherein the adjusted maximum delivery limit is a product of a factor and a basal rate and further wherein the factor is a function of the calculated percentage and a current maximum delivery limit.

14. The method of any one of the preceding claims wherein the percentage of time during a past predetermined time period that a blood glucose reading has indicated hypoglycemia is calculated as a percentage of cycles of the basal dosing algorithm.

15. The method of any one of the preceding claims wherein a higher percentage indicates a downward adjustment of the maximum delivery limit and a lower percentage indicates an upward adjustment of the maximum delivery limit.

FIG. 1

## 200

```
┌─────────────────┐
│  Receive Reading │
│   from CGM       │
│     202          │
└─────────────────┘
         │
         ▼
┌─────────────────┐         ┌─────────────────┐
│ Calculate basal │         │ Calculate Max.  │
│ dose based on   │         │ Delivery Limit  │
│ CGM Reading     │         │     220         │
│     204         │         └─────────────────┘
└─────────────────┘
         │
         ▼
    ◇ Calculated Dose
      <
      Max. Delivery Limit
      ?
      206
```

Calculated Dose < Max. Delivery Limit ? 206

No → Deliver Dose Limited to the Max. Delivery Limit 208

Yes → Deliver Calculated Dose 210

# FIG. 2

**300**

**220**

Calculate % of times
basal dose is limited
by Max. Del. Limit
**302**

↓

Calculate
Adjustment Factor
Based on
Percentage
**304**

↓

Apply Adjustment
to Current Factor
**306** ← Current Factor
(M)

↓

Store Adjusted
Factor as Current
Factor
**308**

↓

Basal Rate → Calculate New
Max. Delivery Limit
**310**

→ Max. Delivery
Limit

## FIG. 3

**400**

**220**

```
Calculate standard
deviation of Set of
BG Readings
402
```

↓

```
Calculate
Adjustment Factor
Based on Standard
Dev.
404
```

↓

```
Apply Adjustment
to Current Factor
406
```  ←  ```
Current Factor
(M)
```

↓

```
Store Adjusted
Factor as Current
Factor
408
```  →  (to Current Factor (M))

↓

```
Basal Rate
```  →  ```
Calculate New
Max. Delivery Limit
410
```

↓

Max. Delivery Limit

*FIG. 4*

_500_

_220_

Calculate % of times
hypoglycemia is
indicated.
_502_

↓

Calculate
Adjustment Factor
Based on
Percentage
_504_

↓

Apply Adjustment
to Current Factor
_506_   ◄———   Current Factor
(M)

↓                ↑

Store Adjusted
Factor as Current
Factor
_508_   ————————————┘

↓

Basal Rate   ———►   Calculate New
Max. Del. Limit
_510_

↓

Max. Delivery
Limit

*FIG. 5*

**600**

**220**

```
        ┌──────────┐
       ╱ Trigger    ╲        No    ┌──────────┐
      ╱  Conditions   ╲─────────────▶│   Exit   │
      ╲  Met?         ╱             └──────────┘
       ╲    602      ╱
        └──────────┘
             │
            Yes
             │
             ▼
    ┌──────────────────┐
    │   Calculate       │
    │ Adjustment Factor │
    │       604         │
    └──────────────────┘
             │
             ▼
    ┌──────────────────┐              ┌──────────────────┐
    │ Apply Adjustment  │◀─────────────│  Current Factor   │
    │ to Current Factor │              │       (M)         │
    │       606         │              │                   │
    └──────────────────┘              └──────────────────┘
             │                                 ▲
             ▼                                 │
    ┌──────────────────┐                       │
    │  Store Adjusted   │───────────────────────┘
    │ Factor as Current │
    │      Factor       │
    │       608         │
    └──────────────────┘
             │
             ▼
    ┌──────────────────┐
    │ Calculate Adjusted│
    │ Nighttime Factor  │
    │       610         │
    └──────────────────┘
             │
             ▼
┌──────────┐    ┌──────────────────┐
│Basal Rate│───▶│  Calculate New    │────▶  Nighttime Max.
│          │    │   Nighttime       │       Delivery Limit
└──────────┘    │ Max. Del. Limit   │
                │       612         │
                └──────────────────┘
```

*FIG. 6*

Europäisches
Patentamt

European
Patent Office

Office européen
des brevets

# EUROPEAN SEARCH REPORT

Application Number

EP 22 17 8984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/066892 A1 (KEENAN DESMOND BARRY [US] ET AL) 6 March 2014 (2014-03-06) * abstract; figure 3 * * paragraph [0185] – paragraph [0188] * * paragraph [0208] – paragraph [0209] * * paragraph [0376] * | 1-15 | INV. G16H20/17 G16H40/63 A61B5/00 A61M5/14 |
| X | WO 2017/209903 A1 (ROCHE DIABETES CARE INC [US]; ROCHE DIABETES CARE INC [US] ET AL.) 7 December 2017 (2017-12-07) * paragraph [0045] – paragraph [0046] * | 1-15 | |
| X | EP 3 443 998 A1 (BIGFOOT BIOMEDICAL INC [US]) 20 February 2019 (2019-02-20) * paragraph [0029] – paragraph [0042] * * paragraph [0064] * * paragraph [0084] * * paragraph [0136] * | 1-15 | |

TECHNICAL FIELDS
SEARCHED        (IPC)

G16H
A61B
A61M

The present search report has been drawn up for all claims

| Place of search | Date of completion of the search | Examiner |
|---|---|---|
| Munich | 19 October 2022 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 8984

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2022

| Patent document cited in search report | | Publication date | Patent family member(s) | | Publication date |
|---|---|---|---|---|---|
| US 2014066892 | A1 | 06-03-2014 | AU | 2013309425 A1 | 26-02-2015 |
| | | | AU | 2015200826 A1 | 12-03-2015 |
| | | | CA | 2882027 A1 | 06-03-2014 |
| | | | CN | 104756116 A | 01-07-2015 |
| | | | CN | 105999479 A | 12-10-2016 |
| | | | EP | 2891087 A2 | 08-07-2015 |
| | | | EP | 2905711 A1 | 12-08-2015 |
| | | | JP | 6239623 B2 | 29-11-2017 |
| | | | JP | 6338553 B2 | 06-06-2018 |
| | | | JP | 2015178044 A | 08-10-2015 |
| | | | JP | 2015526242 A | 10-09-2015 |
| | | | KR | 20150043535 A | 22-04-2015 |
| | | | KR | 20150050562 A | 08-05-2015 |
| | | | US | 2014066884 A1 | 06-03-2014 |
| | | | US | 2014066885 A1 | 06-03-2014 |
| | | | US | 2014066892 A1 | 06-03-2014 |
| | | | US | 2017119968 A1 | 04-05-2017 |
| | | | US | 2020353168 A1 | 12-11-2020 |
| | | | WO | 2014035570 A2 | 06-03-2014 |
| WO 2017209903 | A1 | 07-12-2017 | AU | 2017275393 A1 | 03-01-2019 |
| | | | BR | 112018074909 A2 | 12-03-2019 |
| | | | CA | 3025902 A1 | 07-12-2017 |
| | | | CN | 109478422 A | 15-03-2019 |
| | | | EP | 3479262 A1 | 08-05-2019 |
| | | | KR | 20190013922 A | 11-02-2019 |
| | | | US | 2017348482 A1 | 07-12-2017 |
| | | | WO | 2017209903 A1 | 07-12-2017 |
| EP 3443998 | A1 | 20-02-2019 | AU | 2017207484 A1 | 28-06-2018 |
| | | | AU | 2020294328 A1 | 18-03-2021 |
| | | | AU | 2021204350 A1 | 22-07-2021 |
| | | | AU | 2021204355 A1 | 22-07-2021 |
| | | | CA | 3009351 A1 | 20-07-2017 |
| | | | CN | 108495665 A | 04-09-2018 |
| | | | CN | 112933333 A | 11-06-2021 |
| | | | CN | 113101448 A | 13-07-2021 |
| | | | EP | 3374004 A1 | 19-09-2018 |
| | | | EP | 3443998 A1 | 20-02-2019 |
| | | | EP | 3453414 A1 | 13-03-2019 |
| | | | HK | 1257093 A1 | 11-10-2019 |
| | | | JP | 6876046 B2 | 26-05-2021 |
| | | | JP | 2019509074 A | 04-04-2019 |
| | | | JP | 2021129995 A | 09-09-2021 |
| | | | US | 2017203037 A1 | 20-07-2017 |
| | | | US | 2017203038 A1 | 20-07-2017 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 17 8984

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2022

| Patent document cited in search report | Publication date | Patent family member(s) | | Publication date |
|---|---|---|---|---|
| | | US | 2017203039 A1 | 20-07-2017 |
| | | US | 2017232195 A1 | 17-08-2017 |
| | | US | 2019247578 A1 | 15-08-2019 |
| | | US | 2020376197 A1 | 03-12-2020 |
| | | US | 2021030955 A1 | 04-02-2021 |
| | | US | 2021060245 A1 | 04-03-2021 |
| | | US | 2022031949 A1 | 03-02-2022 |
| | | WO | 2017124006 A1 | 20-07-2017 |

EPO FORM P0459

For more details about this annex : see Official Journal of the European Patent Office, No. 12/82